# EUROPÄISCHE PATENTSCHRIFT

(11) **EP 2 504 334 B1**
(45) Veröffentlichungstag und Bekanntmachung des Hinweises auf die Patenterteilung: **01.10.2014**
(21) Anmeldenummer: 10781686.0
(22) Anmeldetag: 22.11.2010
(51) Int. Cl.: C07D 471/04

(54) **VERFAHREN ZUR REINIGUNG VON METHYL-{4,6-DIAMINO-2-[1-(2-FLUOROBENZYL)-1H-PYRAZOLO[3,4-B]PYRIDIN-3-YL]PYRIMIDIN-5-YL}METHYLCARBAMAT**
PROCESS FOR THE PURIFICATION OF METHYL-{4,6-DIAMINO-2-[1-(2-FLUOROBENZYL)-1H-PYRAZOLO[3,4-B]PYRIDIN-3-YL]PYRIMIDIN-5-YL}METHYLCARBAMATE
PROCÉDÉ DE PURIFICATION DU METHYL-{4,6-DIAMINO-2-[1-(2-FLUOROBENZYL)-1H-PYRAZOLO[3,4-B]PYRIDIN-3-YL]PYRIMIDIN-5-YL}METHYLCARBAMATE

(30) Priorität: 27.11.2009 EP 09177371
(43) Veröffentlichungstag der Anmeldung: 03.10.2012
(60) Teilanmeldung: 13151569.4
(73) Patentinhaber: Bayer Intellectual Property GmbH, 40789 Monheim (DE)
(72) Erfinder: MAIS, Franz-Josef, 40591 Düsseldorf (DE); REHSE, Joachim, 42799 Leichlingen (DE); JOENTGEN, Winfried, 50735 Köln (DE); SIEGEL, Konrad, 40597 Düsseldorf (DE)
(74) Vertreter: BIP Patents
(86) Internationale Anmeldenummer: PCT/EP2010/067949
(87) Internationale Veröffentlichungsnummer: WO 2011/064171

(56) Entgegenhaltungen:
- WO-A1-03/095451
- WO-A1-2005/046725
- WO-A1-2008/031513
- WO-A1-2010/079120
- CHEMMEDCHEM, Bd. 4, Nr. 5, Mai 2009 (2009-05), Seiten 853-865, XP002622814, ISSN: 1860-7179, DOI: DOI:10.1002/CMDC.200900014 in der Anmeldung erwähnt
- EVANS R ET AL: "The Preparation of 4-Amino- and Other Pteridines", JOURNAL OF THE CHEMICAL SOCIETY, CHEMICAL SOCIETY, LETCHWORTH; GB, 1. Januar 1956 (1956-01-01), Seiten 4106-4113, XP009144631, ISSN: 0368-1769
- SCHWOCH S ET AL: "2,3-DIHYDROSPIRO[1H-4- AND 5-AZABENZIMIDAZOLE-2.1'-CYCLOHEXANE] (= SPIRO[CYCLOHEXANE-1,2'(3'H)-1'H-IMIDAZO[4, 5-B]PYRIDINE] AND SPIRO[CYCLOHEXANE-1,2'(3'H)-1'H-IMIDAZO[4, 5-C]PYRIDINE]): REACTIONS WITH NUCLEOPHILES", HELVETICA CHIMICA ACTA, VERLAG HELVETICA CHIMICA ACTA, BASEL, CH, Bd. 77, Nr. 8, 1. Januar 1994 (1994-01-01) , Seiten 2175-2190, XP002073789, ISSN: 0018-019X, DOI: DOI:10.1002/HLCA.19940770811
- BARRACLOUGH P ET AL: "Mono-arylation of 2,3- and 3,4-diaminopyridine and 4,5-diaminopyrimidine, and syntheses of putative inotrope/beta-adrenoceptor antagonists", JOURNAL OF CHEMICAL RESEARCH, SCIENCE REVIEWS LTD, GB, Bd. 9, Nr. 9, 1. Januar 1996 (1996-01-01), Seiten 2316-2335, XP002103810, ISSN: 0308-2342

## Beschreibung

Die vorliegende Erfindung betrifft ein Verfahren zur Reinigung von Methyl-{4,6-diamino-2-[1-(2-fluorobenzyl)-1H-pyrazolo[3,4-b]pyridin-3-yl]pyrimidin-5-yl}methylcarbamat der Formel (I)

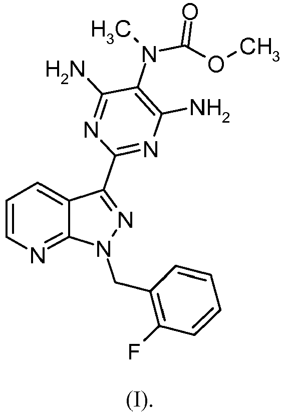

Bei dem Verfahren zur Reinigung des Rohproduktes der Verbindung der Formel (I) für die Verwendung als pharmazeutisch wirksamer Stoff wird Methyl-{4,6-diamino-2-[1-(2-fluorobenzyl)-1H-pyrazolo[3,4-b]pyridin-3-yl]pyrimidin-5-yl}methylcarbamat-sulfinyldimethan (1:1), d.h. eine Verbindung der Formel (II) 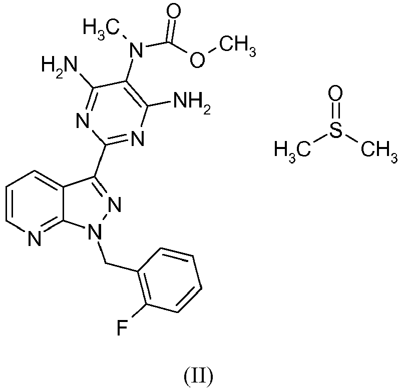 als Zwischenstufe isoliert oder als Zwischenstufe in diesem Reinigungsverfahren, gegebenenfalls in einem Gemisch vorliegend, erzeugt.

Die Verbindung der Formel (I) wirkt als Stimulator der löslichen Guanylatcyclase und kann als Mittel zur Prophylaxe und/oder Behandlung von kardiovaskulären Erkrankungen wie beispielsweise zur Behandlung des Bluthochdrucks und der Herzinsuffizienz, stabiler und instabiler Angina pectoris, peripheren und kardialen Gefäßerkrankungen, von Arrhythmien, zur Behandlung von thromboembolischen Erkrankungen und Ischämien wie Myokardinfarkt, Hirnschlag, transistorischen und ischämischen Attacken, peripheren Durchblutungsstörungen, Verhinderung von Restenosen wie nach Thrombolysetherapien, percutan transluminalen Angioplastien (PTA), percutan transluminalen Koronarangioplastien (PTCA), Bypass sowie zur Behandlung von Arteriosklerose, asthmatischen Erkrankungen und Krankheiten des Urogenitalsystems wie beispielsweise Prostatahypertrophie, erektiler Dysfunktion, weiblicher sexueller Dysfunktion, Osteoporose, Glaukom, pulmonaler Hypertonie, Gastroparese und Inkontinenz eingesetzt werden.

Die Herstellung der Verbindung der Formel (I) und deren Reinigung sind grundsätzlich bekannt. In WO 03/095451 wird die Herstellung der Verbindung der Formel (I) auf folgendem Weg beschrieben.

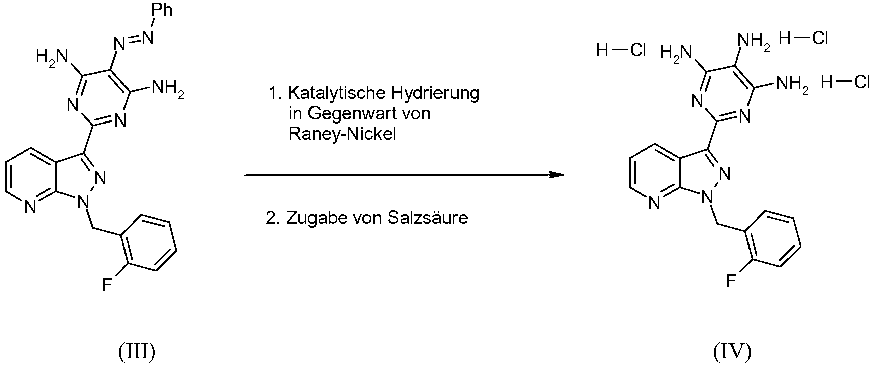

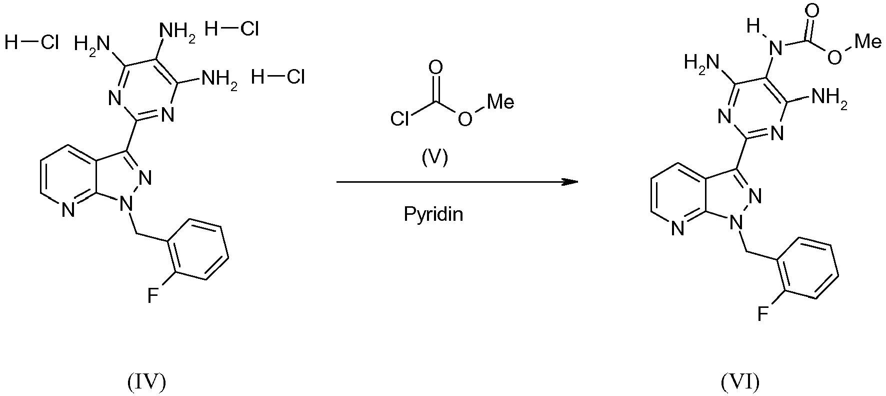

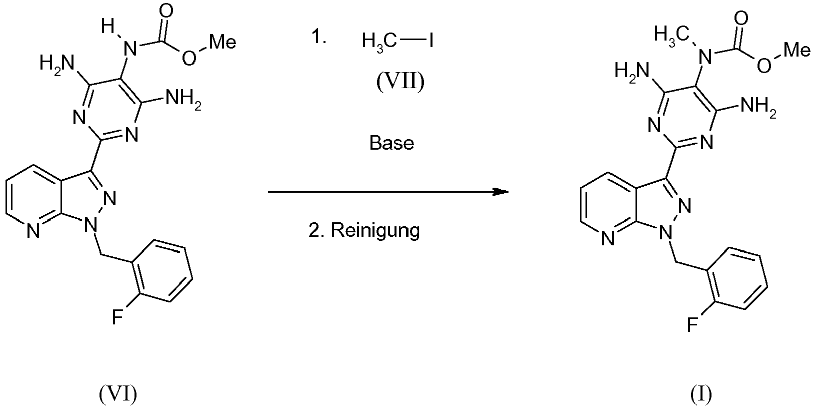

Dabei wird zunächst 2-[1-(2-Fluorbenzyl)-1H-pyrazolo[3,4-b]pyridin-3-yl]-5-[(E)-phenyldiazenyl]pyrimidin-4,6-diamin der Formel (III) durch katalytische Hydrierung gespalten und die resultierende Trisaminoverbindung als 2-[1-(2-Fluorbenzyl)-1H-pyrazolo[3,4-b]pyridin-3-yl]-4,5,6-pyrimidintriamin Trihydrochlorid der Formel (IV) isoliert. Dieses Trihydrochlorid wird dann mit Chlorameisensäuremethylester der Formel (V) zu Methyl-4,6-diamino-2-[1-(2-fluorbenzyl)-1H-pyrazolo[3,4-b]pyridin-3-yl]-5-pyrimidinylcarbamat der Formel (VI) in Pyridin als Lösungsmittel umgesetzt. Alternativ wird in ChemMedChem 2009, 4, 853-865 beschrieben, daß die Trisaminoverbindung als Trihydrochlorid isoliert wird und anschließend die HCl freie Base durch Ausschütteln mit wäßriger NaHCO₃-Lösung erzeugt und die freie Base mit Chlorameisensäuremethylester der Formel (V) zur Verbindung der Formel (VI) in Pyridin als Lösungsmittel umgesetzt wird. Dann wird die Verbindung der Formel (VI) mit Methyliodid der Formel (VII) in Gegenwart einer Base zum Rohprodukt der Verbindung der Formel (I) umgesetzt. Die Reinigung des Rohproduktes der Verbindung der Formel (I) erfolgt nach der experimentellen Vorschrift des Beispiels 8 der WO 03/095451 und der vergleichbaren Beschreibung in ChemMedChem 2009, 4, 853-865 durch Ausrühren des Rohproduktes in Dichlormethan/THF, Zwischenisolierung des Dichlormethan/THF ausgerührten Produktes durch Filtration, Auskochen des isolierten Feststoffs mit Methanol, Zwischenisolierung des mit Methanol ausgekochten Festsstoffs durch Filtration, Auflösung des Festsstoffs in einer Mischung von Dioxan, Dichlormethan und Methanol in Gegenwart von Aktivkohle, Filtration der Aktivkohle über Kieselgur bzw. Celite, Einengen der filtrierten Lösung bis zur Trockene, Ausrühren des zur Trockene eingeengten Feststoffs mit Methanol, Isolierung des Methanol ausgerührten Festsstoffs durch Filtration und (nicht im WO 03/0945451 in Beispiel 8 oder ChemMedChem 2009, 4, 853-865 beschrieben aber objektiv notwendig) Trocknung. Alternativ ist eine Reinigung eines zur Trockene eingeengten Rohproduktes der Verbindung der Formel (I) durch präparative Chromatographie (RP-HPLC) in schlechten Ausbeuten möglich.

Diese Synthese und die Reinigungen besitzen eine Reihe von Nachteilen, die für eine technische Durchführung in großem Maßstab sehr ungünstig sind. Besonders nachteilig sind die Reinigungsverfahren des Rohproduktes der Formel (I). Eine effektive Reinigung ist für die Verwendung als pharmazeutischer Wirkstoff zwingend notwendig. Die beschriebene Reinigung über RP HPLC, d.h. die chromatographische Reinigung stellt eine Labormethode dar, die im technischen Maßstab nur sehr aufwendig durchführbar ist. Zudem ist die angegebene Ausbeute von nur 29% für den Syntheseschritt zum Rohprodukt der Formel (I) und dessen Reinigung sehr niedrig. Die alternative Herstellungs- und Reinigungsmethode ist sehr umständlich. Sie enthält insgesamt 5 Feststoffisolierungen (2 Einengungen zur Trockene und 3 Filtrationen) wobei, wie bereits oben erwähnt, Einengungen zur Trockene im technischen Maßstab sehr ungünstig sind. Insgesamt ist bei der Durchführung einer chemischen Stufe eine Zahl von 5 Feststoffisolierungen für die Herstellung und Reinigung eines pharmazeutischen Wirkstoffs im technischen Maßstab sehr nachteilig.

Es bestand daher Aufgabe ein vereinfachtes Verfahrens zu finden, das sicher und auch im großtechnischen Maßstab vorteilhaft durchgeführt werden kann und das einen Wirkstoff in hoher Ausbeute und hoher Reinheit im pharmazeutisch akzeptabler Qualität liefert.
Überraschend wurde nun ein Verfahren zur Reinigung von Methyl{4,6-diamino-2-[1-(2-fluorobenzyl)-1H-pyrazolo[3,4-b]pyridin-3-yl]pyrimidin-5-yl}methylcarbamat der Formel (I) 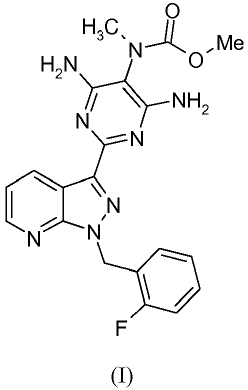 für seine Verwendung als pharmazeutischer Wirkstoff gefunden. Dieses neue Verfahren unterscheidet sich von den bislang bekannten Verfahren im folgenden Punkt:

Die Reinigung des Rohproduktes der Formel (I) für die Verwendung als pharmazeutischer Wirkstoff erfolgt über die Verbindung Methyl-{4,6-diamino-2-[1-(2-fluorobenzyl)-1H-pyrazolo[3,4-b]pyridin-3-yl]pyrimidin-5-yl}methylcarbamat-sulfinyldimethan (1:1), d.h. eine Verbindung der Formel (II) als isolierte Zwischenstufe oder welche in einem Gemisch erzeugt wird

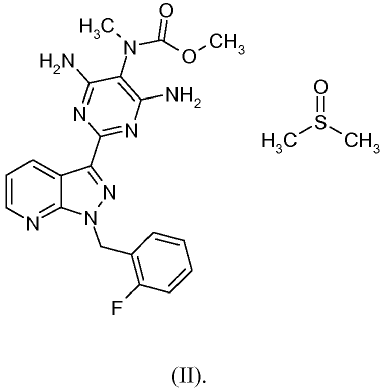

Dieser Unterschied ermöglicht die Überwindung der Nachteile der bislang bekannten Verfahren und einen Wirkstoff in hoher Ausbeute und hoher Reinheit in pharmazeutisch akzeptabler Qualität.

Im folgenden wird das erfindungsgemäße Verfahren zur Reinigung der Verbindung der Formel (I) über das Zwischenprodukt der Formel (II) genau beschrieben.

### Reinigung des Rohproduktes der Verbindung der Formel (I)

Das Rohprodukt der Formel (I) wird erfindungsgemäß für die Verwendung als pharmazeutischer Wirkstoff gereinigt. Dazu wird zunächst ein Gemisch erzeugt, das die Verbindung der Formel (II) als Zwischenprodukt in hohen Anteilen enthält.

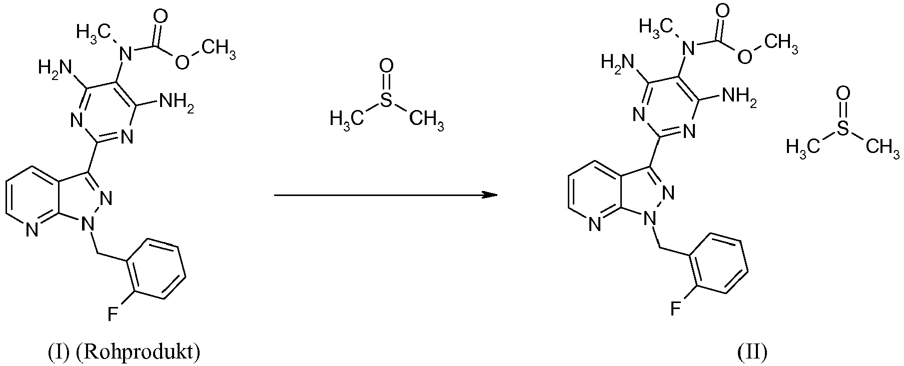

Dazu wird das Rohprodukt der Formel (I) in DMSO gegebenenfalls in Gegenwart eines pharmazeutisch akzeptablen einfachen Lösungsmittels aus der Klasse der Ketone, Ether, Ester oder Alkohole gelöst. Als Beispiele für solche Lösungsmittel seien genannt: Methanol, Ethanol, Isopropanol, 1-Butanol, 2-Butanol, Ethylacetat, Isopropyl- oder Propylacetat, Butylacetat, tert.-Butylmethylether, Diisopropylether, Aceton, Methylethylketon, Methylisobutylketon usw. Bevorzugt sind Ethanol, Isopropanol, Ethylacetat, Isopropylacetat, Butylacetat, Methylethylketon, Methylisobutylketon, besonders bevorzugt ist Ethylacetat. Es können auch Gemische dieser Lösungsmittel eingesetzt werden.

DMSO wird zugegeben in einer Menge von 100 bis 750 Gew.-% auf die eingesetzte Menge des Rohproduktes der Formel (I), bevorzugt 150 bis 500 Gew.-%.

Gegebenenfalls kann zu dieser Mischung Aktivkohle zugesetzt werden in einer Menge von 0,25 bis 35 Gew.-% auf die eingesetzte Menge des Rohproduktes der Formel (I), bevorzugt 0,5 bis 20% Gew.-%.

Zur Erzeugung einer Lösung wird das Gemisch erhitzt auf 40-120°C, bevorzugt auf 50-100°C.

Zur Erzeugung eines pharmazeutisch akzeptablen Produktes der Formel (I) muß die Lösung filtriert werden. Die Filtration muß unabhängig davon durchgeführt werden ob Aktivkohle zugesetzt wurde oder nicht.

Die Menge des pharmazeutisch akzeptablen Lösungsmittels das neben DMSO zur Lösung des Rohproduktes der Formel (I), d.h. vor der Filtration eingesetzt wird beträgt 25 bis 200 Gew. % bezogen auf DMSO, bevorzugt 40 bis 100 Gew.-%.

Die Filtration wird in der Hitze durchgeführt, die Temperaturen sind 40-120°C, bevorzugt 50-100°C.

Nach der Filtration wird in der Hitze ein pharmazeutisch akzeptables Lösungsmittel zugesetzt, bevorzugt das gleiche Lösungsmittel wie zuvor. Dadurch wird das Produkt der Formel (II) zur Kristallisation gebracht.

Die Gesamtmenge Menge des vor und nach der Filtration zugesetzten Lösungsmittels beträgt 200 bis 1500 Gew.-% bezogen auf DMSO, bevorzugt 400-1200 Gew.-%.

Die Temperatur der Zugabe liegt bei 30-110°C, bevorzugt 35-90°C.

Vor der Isolierung des Feststoffes, der die Verbindung der Formel (II) zu hohen Anteilen enthält wird zur Vervollständigung der Fällung abgekühlt in einen Temperaturbereich von 0-35°C, bevorzugt auf Normaltemperatur von z.B. 20-30°C.

Die Isolierung wird über übliche Isolieraggregate wie Nutsche oder Zentrifuge durchgeführt. Zur Entfernung der Mutterlauge wird das isolierte Material bei der Isolierung mit einem pharmazeutisch akzeptablen Lösungsmittel gewaschen, bevorzugt ist das gleiche Lösungsmittel wie zuvor.

Das isolierte Material nach der DMSO-Umlösung enthält zu hohen Anteilen das Produkt der Formel (II). Daneben kann das Produkt der Formel (I) üblicherweise zu geringeren Anteilen auch direkt ausfallen ohne ein Solvat mit DMSO zu bilden. Es ist weiterhin auch die Bildung von Solvaten anderer Stöchiometrie oder die Bildung von Lösungsmitteladdukten ohne genau festgelegte Stöchiometrie möglich. Zudem kann auch DMSO in nicht gebundener Form als anhaftender Lösungsmittelrest enthalten sein. Der Gehalt an DMSO in dem isolierten Material liegt üblicherweise bei 10 bis 25 Gew.-%, bevorzugt bei 12-17%. Es ist erfindungsgemäß besonders bevorzugt, das Produkt der Formel (II) in Form dieses Gemisches zu erzeugen und für die Herstellung des gereinigten Produktes der Formel (I) zu benutzen

Das so erhaltene Produkt der Formel (II) kann nun getrocknet werden oder auch in feuchter Form mit Gehalt an Restlösungsmitteln, d.h. anhaftendes DMSO und das bzw. die Fällungslösungsmittel in die Umwandlung zum gereinigten Produkt der Formel (I) eingesetzt werden.

Die Verbindung der Formel (II) ist neu. Sie kann wie in bei den weiter unten folgenden Ausführungsbeispielen beschrieben ist, in reiner Form hergestellt werden und analytisch charakterisiert werden.

Für eine pharmazeutische Verwendung muß aus dem Produkt der Formel (II) oder Gemisch, das die Verbindung der Formel (II) zu hohen Anteilen enthält das DMSO entfernt werden.

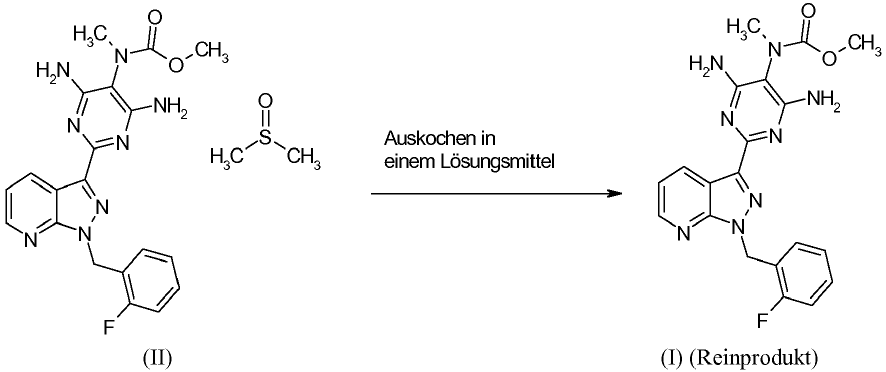

Dazu wird das Produkt der Formel (II) oder das isolierte Gemisch, das das Produkt der Formel (II) in hohen Anteilen enthält in einem pharmazeutisch akzeptablen Lösungsmittel aus der Klasse der Ketone, Ether, Ester oder Alkohole ausgekocht. Als Beispiele für solche Lösungsmittel seien genannt: Methanol, Ethanol, Isopropanol, 1-Butanol, 2-Butanol, Ethylacetat, Isopropyl- oder Propylacetat, Butylacetat, tert.-Butylmethylether, Diisopropylether, Aceton, Methylethylketone, Methylisobutylketon usw. Bevorzugt sind Ethanol, Isopropanol, Ethylacetat, Isopropylacetat, Butylacetat, Methylethylketon, Methylisobutylketon. Es können auch Gemische dieser Lösungsmittel eingesetzt werden. Besonders bevorzugt ist Ethylacetat oder ein Gemisch aus Ethylacetat mit Ethanol.

Die Auskochung wird am Rückfluß des jeweiligen Lösungsmittels oder gegebenenfalls bei leicht erhöhtem Druck durchgeführt. Die Temperatur ist 50-150°C, bevorzugt 80-120°C.

Das erfindungsgemäße Verfahren bietet deutliche Vorteile gegenüber dem Stand der Technik. Vor allem überraschend ist, daß die Reinigung des Rohproduktes der Formel (I) für eine pharmazeutische Verwendung besonders aus einer Umlösung mit einem DMSO-haltigen Lösungsmittelgemisch erfolgt und daß dabei die neue Verbindung der Formel (II) als Zwischenstufe gegebenenfalls in einem Gemisch in hohen Anteilen erhalten wird. Durch diesen Schritt werden sämtliche Verunreinigungen bis auf niedrige Restmengen abgetrennt, so daß man nach der Entfernung des DMSO Gehaltes durch einfaches Auskochen einen hochreinen Feststoff der Formel (I) zurück behält. Dieser Feststoff ist der Regel farblos bis ganz leicht gelb und die analytische Reinheit (HPLC) liegt deutlich über 98 Gew.-% was für eine pharmazeutische Verwendung sehr vorteilhaft ist.

Das Verfahren ist technisch sicher durchführbar und es erlaubt eine Produktion im großtechnischen Maßstab. Es läßt sich flexibel an betriebliche apparative Voraussetzungen anpassen. Eine besonders bevorzugte Ausführungsform ist, daß bei der Reinigung des Rohproduktes der Formel (I) die Zwischenisolierung des Produktes der Formel (II) oder des Gemisches, das die Verbindung der Formel (II) zu hohen Anteilen enthält in einem Nutschtrockner durchgeführt wird. Die folgende Entfernung des DMSO aus dem im Nutschtrockner zwischenisolierten Produkt der Formel (II) erfolgt durch direkte Zugabe von Lösungsmittel in den Nutschtrockner mit oder ohne Zwischentrocknung des Produktes der Formel (II). Dadurch wird ein offenes Handling des Feststoffs des Produktes der Formel (II) mit der Gefahr von Verunreinigung vermieden.

### Experimentller Teil

### Abkürzungen und Akronyme:

- abs.: absolut
- cat.: katalytisch
- CI: chemische Ionisation (bei MS)
- d: Tag(e)
- DC: Dünnschichtchromatographie
- DMF: Dimethylformamid
- DMSO: Dimethylsulfoxid
- d. Th.: der Theorie (bei Ausbeute)
- ee: Enantiomerenüberschuss
- EI: Elektronenstoß-Ionisation (bei MS)
- ent: Enantiomer / enantiomerenrein
- eq: Äquivalent(e)
- ESI: Elektrospray-Ionisation (bei MS)
- GC-MS: Gaschromatographie-gekoppelte Massenspektrometrie
- Gew.-%: Gewichtsprozent
- h: Stunde(n)
- HPLC: Hochdruck-, Hochleistungsflüssigchromatographie
- konz.: konzentriert
- LC-MS: Flüssigchromatographie-gekoppelte Massenspektrometrie
- min: Minute(n)
- MS: Massenspektrometrie
- NMR: Kernresonanzspektrometrie
- Ph: Phenyl
- R_{f}: Retentionsindex (bei DC)
- Rₜ: Retentionszeit (bei HPLC)
- RT: Raumtemperatur
- v/v: Volumen-zu-Volumen-Verhältnis (einer Lösung)
- wässr.: wässrig, wässrige Lösung

### Die folgenden Beispiele erläutern die Erfindung ohne Sie jedoch darauf einzuschränken.

### Referenz Beispiel 1

### Herstellung von 2-[1-(2-Fluorbenzyl)-1H-pyrazolo[3,4-b]pyridin-3-yl]-4,5,6-pyrimidintriamin (VIII)

In einem Druckautoklaven wurden 1100g der Verbindung der Formel (III) in 5,41 DMF suspendiert. Man gab 44g eines handelsüblichen wasserfeuchten (ca. 50%ig) 5%Pd-Kohle Katalysators zu und der verschlossene Autoklav wurde nach Inertisierung mit Stickstoff und Aufdrücken von Wasserstoff ca. 18h bei 65bar Wasserstoff und ca. 60°C Innentemperatur hydriert. Nach dem Abkühlen auf ca. 25°C, Entspannen und Inertisieren wurde der Autoklaveninhalt ausgenommen, wobei mit 650ml DMF nachgespült wurde.

Drei solcher gleichartig durchgeführter Ansätze wurden vereinigt, der Altkatalysator wurde ab filtriert, man spülte mit 1,11 DMF und das Filtrat wurde im Vakuum auf ca. ein Drittel seiner Masse eingeengt. In den Rückstand von ca. 6,5kg wurden nacheinander 8,251 Methanol und 8,251 Wasser eindosiert, die Suspension wurde zur Vervollständigung der Kristallisation auf ca. 5°C abgekühlt, der Feststoff wurde abfiltriert und mit Methanol/Wasser (1:1 Vol) gewaschen. Das Produkt wurde bei 50°C im Vakuum getrocknet. Die Auswaage betrug 2415g entsprechend 91,8% d.Th. Der Gehalt des Zielproduktes der Formel (VIII) (freie Base) betrug >98 Flächen-% bzw. >97 Gew.-%. Die größten Verunreinigungen waren DMF (ca. 0,8 Gew. %) und Wasser (ca. 0,5 Gew.-%).

### Referenz Beispiel 2

### Herstellung von Methyl-4,6-diamino-2-[1-(2-fluorbenzyl)-1H-pyrazolo[3,4-b]pyridin-3-yl]-5-pyrimidinylcarbamat (VI)

In einem Reaktionsgefäß wurden 3063g der Verbindung der Formel (VIII) und 30,71 techn. Isopropanol vorgelegt. Dazu dosierte man unter Rühren 1641g Dimethyldicarbonat bei 20-25°C ein und rührte 22h bei dieser Temperatur nach. Das ausgefallene Produkt wurde abgesaugt, mit techn. Isopropanol gewaschen und im Vakuum bei 50°C getrocknet. Man erhielt eine Auswaage von 3748g bzw. 105,9% d.Th. Das Produkt der Formel (I) enthielt unter anderem ca. 4,7% praktisch nicht durch Trocknung entfernbares Isopropanol (es lag teilweise ein Isopropanol-Solvat vor) und der analytische Gehalt lag bei 89,5 Gew.-% (HPLC). Bezogen auf diesen Gehalt lag die Ausbeute bei 94,8% d.Th.

### Referenz Beispiel 3

### Herstellung von 2-[1-(2-Fluorbenzyl)-1H-pyrazolo[3,4-b]pyridin-3-yl]-4,5,6-pyrimidintriamin (VIII)

In einem Druckautoklaven wurden 300g der Verbindung der Formel (III), 1600ml DMF und 60g wasserfeuchtes Raney-Nickel vorgelegt und nach Inertisierung bei 60°C Innentemperatur, 65bar Wasserstoff für ca. 18h hydriert. Nach Abkühlen und Entspannen wurde der Altkatalysator ab filtriert und mit 100ml DMF gespült. Das Filtrat wurde auf 534,5g im Vakuum eingeengt und zu dem Rückstand dosierte man bei 35-40°C 750ml Methanol und dann nach Abkühlen bei 0-5°C 750ml Wasser ein. Der Feststoff wurde filtriert und bei 50°C im Vakuum getrocknet. Die Auswaage betrug 219,7g bzw. 91,8% d.Th.

### Referenz Beispiel 4

### Herstellung von Methyl-4,6-diamino-2-[1-(2-fluorbenzyl)-1H-pyrazolo[3,4-b]pyridin-3-yl]-5-pyrimidinylcarbamat (VI)

In einem Reaktionsgefäß wurden 1,50kg der Verbindung der Formel (VIII) in 14,251 Isopropanol vorgelegt und unter Rühren auf 35°C erhitzt. Dazu dosierte man 531g Chlorameisensäuremethylester in 30min gleichmäßig schnell ein, spülte mit 750ml Isopropanol nach und rührte 16h bei 35°C nach. Dann erhitzte man auf 50°C, dosierte 3,851 Methanol und 606g Triethylamin bei 50°C unter Rühren ein und spülte mit 450ml Methanol nach. Dann rührte man 1h bei 50°C nach, kühlte auf RT ab und rührte bei RT 1h nach. Der suspendierte Feststoff wurde abgesaugt, mit je 3,01 Isopropanol/Methanol (4:1) zweimal und einmal mit 3,01 Isopropanol gewaschen und trocken gesaugt. Das Feuchtprodukt wurde bei 50°C für 1h und anschließend bei 100°C für 22h im Vakuumtrockenschrank getrocknet. Man erhielt eine Auswaage von 1,793kg bzw. 103,3% d.Th. Das Produkt der Formel (VI) enthielt 6,45% praktisch nicht durch Trocknung entfernbares Isopropanol (es lag teilweise ein Isopropanol-Solvat vor) und der analytische Gehalt lag bei 87,9 Gew.-% (HPLC). Bezogen auf diesen Gehalt lag die Ausbeute bei 90,8% d.Th

### Vergleichsbeispiel 5

### Herstellung von Methyl-4,6-diamino-2-[1-(2-fluorbenzyl)-1H-pyrazolo[3,4-b]pyridin-3-yl]-5-pyrimidinyl(methyl)carbamat (I)

### (Methylierung in an sich bekannter Weise nach WO 03/095451, Bespiel 8 zweite Vorschrift)

1630g der Verbindung der Formel (VI) wurden bei 20-25°C in 16,31 THF suspendiert. Man kühlte auf -6 bis -4°C ab und dosierte 3480g einer 1M Lösung von Bis(trimethylsilyl)natriumamid ein. Man rührte nach und dosierte 596g Methyliodid ein, rührt kurz nach und ließ langsam auf ca. 5°C aufwärmen. Man rührte bei dieser Temperatur bis die Umsetzung beendet war (ca. 4h). Die Reaktionsmischung wurde mit 4 mal 4,11 15% Ammoniumchlorid-Lösung gewaschen. Die organische Phase wurde auf ca. 6.4kg Rückstand eingedampft und auf ca. 25°C temperiert. Der ausgefallene Feststoff wurde abfiltriert, mit insgesamt 31 THF gewaschen und im Vakuum bei 50°C getrocknet. Man erhielt 1112g an dem Rohprodukt der Formel (I). Das waren 75,2% der Theorie an Ausbeute.

### Beispiel 6

### Herstellung eines Gemisches bestehend aus Methyl-4,6-diamino-2-[1-(2-fluorbenzyl)-1H-pyrazolo[3,4-b]pyridin-3-yl]-5-pyrimidinyl(methyl)carbamat (I) und Methyl-{4,6-diamino-2-[1-(2-fluorobenzyl)-1H-pyrazolo[3,4-b]pyridin-3-yl]pyrimidin-5-yl}methylcarbamat-sulfinyldimethan (II) mit hohen Anteilen des Produktes der Formel (II)

9,0g eines Rohproduktes der Formel (I), das in vergleichbarer Weise zu Vergleichsbeispiel 5 hergestellt wurde wurden in 16ml DMSO bei 100°C gelöst. (Die Klärfiltration, die zur Erzeugung einer pharmazeutisch akzeptablen Produktqualität an dieser Stelle notwendig gewesen wäre wurde bei diesem Laborversuch weggelassen) Dann ließ man auf 75°C abkühlen, gab 110ml Ethylacetat hinzu und kühlte langsam auf ca. 25°C ab. Der ausgefallene Feststoff wurde abfiltriert, mit insgesamt 28ml Ethylacetat gewaschen und bei 50°C im Vakuum getrocknet. Die Auswaage betrug 9,6g bzw. 90,0% d.Th.

### Beispiel 7

### Herstellung von gereinigtem Methyl-4,6-diamino-2-[1-(2-fluorbenzyl)-1H-pyrazolo[3,4-b]pyridin-3-yl]-5-pyrimidinyl(methyl)carbamat (I)

Die gesamte Menge des im obigen Beispiel 6 hergestellten Produktes der Formel (II) wurde in 135ml Ethylacetat 1h am Rückfluß (ca. 78°C) gerührt und auf ca. 25°C gekühlt. Der Feststoff wurde abgesaugt, mit insgesamt 36ml Ethylacetat gewaschen und im Vakuum getrocknet. Die Auswaage betrug 7,6g bzw. 93,8% d.Th. Der Gehalt des Produktes lag deutlich über 98 Gew.-% (HPLC). Als Lösungsmittel war Ethylacetat in einer Menge von ca. 0,2% enthalten. Der DMSO Gehalt lag unter 0,1 %.

### Beispiel 8

### Herstellung von gereinigtem Methyl-4,6-diamino-2-[l-(2-fluorbenzyl)-1H-pyrazolo[3,4-b]pyridin-3-yl]-5-pyrimidinyl(methyl)carbamat (I) mit Zwischenisolierung eines Gemisches mit hohen Anteilen an Methyl-{4,6-diamino-2-[1-(2-fluorobenzyl)-1H-pyrazolo[3,4-b]pyridin-3-yl]pyrimidin-5-yl}methylcarbamat-sulfinyldimethan (II) als Feuchtprodukt

193,5g eines Rohproduktes der Formel (I), das in vergleichbarer Weise zu Vergleichsbeispiel 5 hergestellt wurde, wurden in 344ml DMSO und 172ml Ethylacetat bei ca. 96°C gelöst. Dann wurden 19,4g Aktivkohle und 172ml Ethylacetat zugegeben und in der Hitze gerührt. Dann wurde die Aktivkohle in der Hitze filtriert und mit 172ml Ethylacetat nachgespült. Das Filtrat wurde auf 78°C temperiert und langsam mit 1850ml Ethylacetat versetzt. Die Mischung wurde in ca. 2-3h auf ca. 25°C abgekühlt, der Feststoff wurde abfiltriert und mit insgesamt 772ml Ethylacetat gewaschen. Das Feuchtprodukt, das hohe Anteile der Verbindung der Formel (II) im Gemisch enthielt, wurde in 2900ml Ethylacetat suspendiert, auf 1h Rückfluß erhitzt und auf ca. 25°C abgekühlt. Der Feststoff wurde abgesaugt, mit insgesamt 774ml Ethylacetat gewaschen und im Vakuum bei 50°C getrocknet. Man erhielt 155,1g an Auswaage bzw. 80,2% vom Einsatz. Der Gehalt des Produktes lag deutlich über 98 Gew.-% (HPLC). Als Lösungsmittel waren praktisch nur Ethylacetat und DMSO in geringer Menge enthalten.

### Beispiel 9

### Herstellung und analytische Charakterisierung von Methyl-{4,6-diamino-2-[1-(2-fluorobenzyl)-1H-pyrazolo[3,4-b]pyridin-3-yl]pyrimidin-5-yl}methylcarbamat-sulfinyldimethan (II)

14,8g eines Rohproduktes der Formel (I), das in vergleichbarer Weise zu Vergleichsbeispiel 5 hergestellt wurde, wurden in 28,9g DMSO und 11,85g Ethylacetat bei ca. 94°C gelöst. Dann gab man 1,5g Aktivkohle Norit A-Supra und weitere 11,85g Ethylacetat zu, rührte 1h bei Rückfluß (88-90°C) nach und filtrierte die Aktivkohle heiß ab. Der zum Teil bereits ausgefallene Feststoff wurde durch Erwärmen bis ca. 78°C wieder zur Lösung gebracht und die Lösung wurde anschließend langsam abkühlen lassen. Der ausgefallenen Feststoff wurde bei RT abgesaugt, dreimal mit je 50ml Ethylacetat gewaschen und 18h bei 30°C im Trockenschrank getrocknet. Man erhielt 9,2g bzw. 52,5% d.Th eines leicht gelblichen Kristallpulvers der Verbindung der Formel (II).
HPLC: 99,90 F1.-% (ohne Berücksichtigung des DMSO)
DMSO (GC): 14,7 Gew.-%
¹H-NMR (400MHz in DMF-d₇):
d = 2,59 (s, ca. 6H, 2 CH₃ an DMSO), 3,13 (s, 3H, N-CH₃), 3,58 + 3,67 (zwei s, 3H, gehinderte Rotation an O-CH₃), 5,91 (s ,2H, -CH₂-), 6,53 (s, 4H, 2 -NH₂), 7,05-7,40 (m, 5H, 4 aromatische H am o-Fluorbenzyl Substituenten und 1H am Pyridoring meta zum Pyrido-Stickstoff), 8,60 (dd, 1H, am Pyridoring ortho zum Pyrido-Stickstoff), 9,12 (dd, 1H, am Pyridoring para zum Pyrido-Stickstoff).

### Elementaranalyse:

| | | | |
|---|---|---|---|
| gefunden | C: 52,2% | berechnet | C: 52,79% |
| | H: 4,9% | | H: 5,03% |
| | N: 22,7% | | N: 22,39% |

## Patentansprüche

1. Verfahren zur Reinigung von Methyl-{4,6-diamino-2-[1-(2-fluorobenzyl)-1H-pyrazolo[3,4-b]pyridin-3-yl]pyrimidin-5-yl}methylcarbamat der Formel 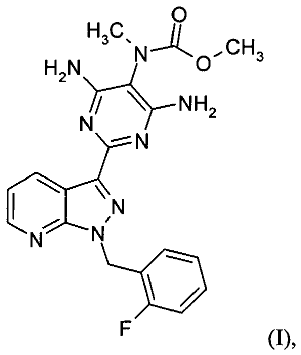 **dadurch gekennzeichnet, dass** das Rohprodukt der Verbindung der Formel (I) in Dimethylsulfoxid gelöst und das dabei anfallende Methyl-{4,6-diamino-2-[1-(2-fluorobenzyl)-1H-pyrazolo[3,4-b]pyridin-3-yl]pyrimidin-5-yl}methylcarbamat-sulfinyldimethan der Formel 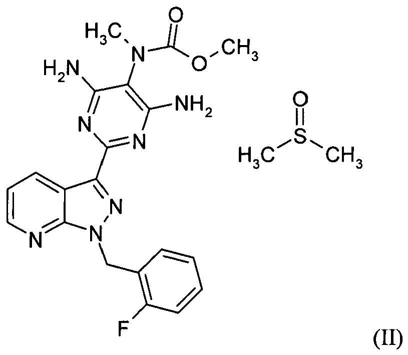 isoliert und das Dimethylsulfoxid anschliessend durch auskochen in einem pharmazeutisch akzeptablen Lösungsmittel wieder entfernt wird.

2. Methyl-{4,6-diamino-2-[1-(2-fluorobenzyl)-1H-pyrazolo[3,4-b]pyridin-3-yl]pyrimidin-5-yl}methylcarbamat-sulfinyldimethan der Formel

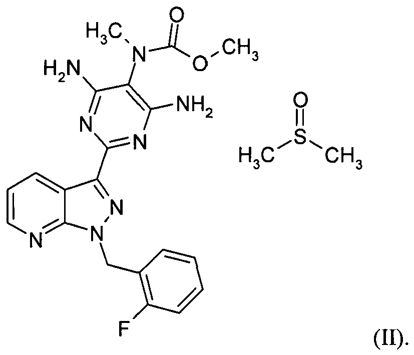

## Claims

1. Process for purifying methyl {4,6-diamino-2-[1-(2-fluorobenzyl)-1H-pyrazolo[3,4-b]pyridin-3-yl]pyrimidin-5-yl}methylcarbamate of the formula 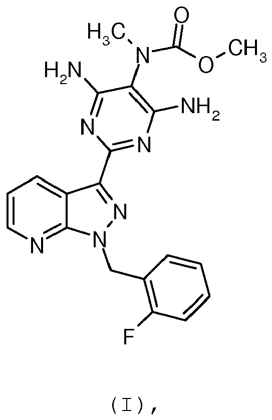 **characterized in that** the crude product of the compound of the formula (I) is dissolved in dimethyl sulphoxide and the resulting methyl {4,6-diamino-2-[1-(2-fluorobenzyl)-1H-pyrazolo[3,4-b]pyridin-3-yl]pyrimidin-5-yl}methylcarbamate sulphinyldimethane of the formula 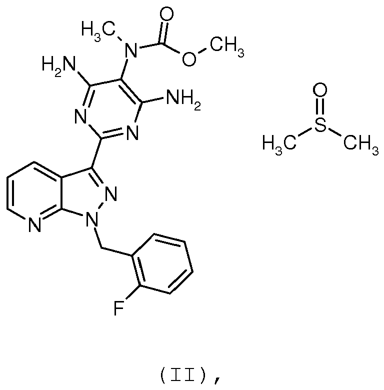 is isolated and the dimethyl sulphoxide is subsequently removed again by boiling in a pharmaceutically acceptable solvent.

2. Methyl {4,6-diamino-2-[1-(2-fluorobenzyl)-1H-pyrazolo[3,4-b]pyridin-3-yl]pyrimidin-5-yl}methylcarbamate sulphinyldimethane of the formula

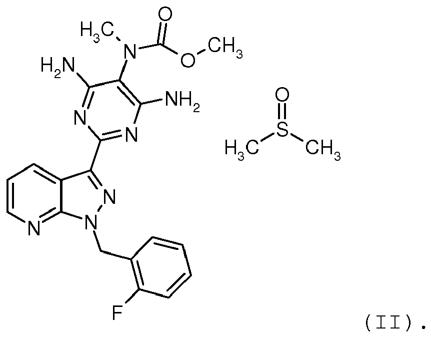

## Revendications

1. Procédé de purification de carbamate de méthyl-{4,6-diamino-2-[1-(2-fluorobenzyl)-1H-pyrazolo[3,4-b]pyridin-3-yl]pyrimidin-5-yl}méthyle de formule 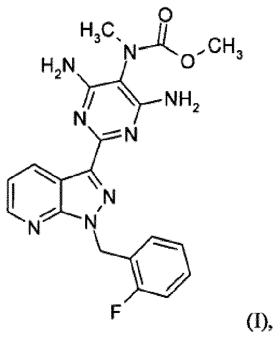 **caractérisé en ce que** le produit brut du composé de formule (I) est dissous dans du sulfoxyde de diméthyle et le carbamate de méthyl-{4,6-diamino-2-[1-(2-fluorobenzyl)-1H-pyrazolo[3,4-b]pyridin-3-yl]pyrimidin-5-yl}méthyle-sulfinyldiméthane formé de formule 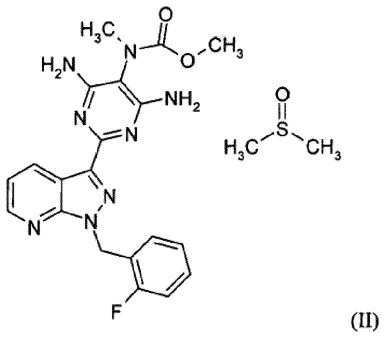 est isolé, puis le sulfoxyde de diméthyle est éliminé par ébullition dans un solvant pharmaceutiquement acceptable.

2. Carbamate de méthyl-{4,6-diamino-2-[1-(2-fluorobenzyl)-1H-pyrazolo[3,4-b]pyridin-3-yl]pyrimidin-5-yl}méthyle-sulfinyldiméthane de formule
